# EUROPEAN PATENT SPECIFICATION

(11) **EP 0 390 329 B1**
(45) Date of publication and mention of the grant of the patent: **01.12.1993**
(21) Application number: 90301999.0
(22) Date of filing: 26.02.1990
(51) Int. Cl.: C09K 19/34, C07D 239/26

(54) **Pyrimidine derivative**
Pyrimidin-Derivate
Dérivés pyrimidiques

(30) Priority: 30.03.1989 JP 79034/89; 05.07.1989 JP 173511/89
(43) Date of publication of application: 03.10.1990
(73) Proprietor: SEIKO EPSON CORPORATION, Shinjuku-ku Tokyo (JP)
(72) Inventor: Yamada, Shuhei, c/o Seiko Epson Corporation, Suwa-shi, Nagano-ken (JP)
(74) Representative: Miller, Joseph

(56) References cited:
- EP-A- 0 317 175
- WO-A-86/04081

## Description

This invention relates to pyrimidine derivatives, for example, for use as electro-optical display material.

According to WO-A-86 04 081, it is known to use 4-cyano-3-fluorophenyl-pyrimidine derivatives as electro-optical display material.

According to the present invention, there is provided a pyrimidine derivative of the general formula: 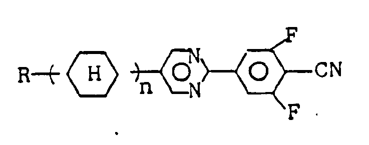
where R is a straight chain alkyl group with 1 to 10 carbon atoms, n is 0 or 1 and the cyclohexane ring is in the trans form.

Compounds according to the present invention have a relatively large and positive anisotropic dielectric constant Δε. Accordingly, a liquid crystal display device which needs only an extremely low driving voltage can be constructed by using a liquid crystal composition comprising a mixture of a pyrimidine compound according to the present invention and at least one other liquid crystal compound.

A liquid crystal display device utilises the electro-optical effect of liquid crystal material and a liquid crystal phase used therefor includes the nematic phase, the cholesteric phase and the smectic phase. The display system employed most generally at present is the twisted nematic (hereinafter simply referred to as TN) mode utilising the nematic phase.

Liquid crystal display devices have merits such as:
1. Small size and reduced thickness;
2. Low driving voltage and reduced power consumption; and
3. Being passive display devices, no eye fatigue even after long periods of use.

Accordingly, liquid crystal display devices are used in, for example, portable electronic computers, audio equipment, various instruments, automobile dash boards, etc. In particular, liquid crystal display devices have recently been used as displays for personal computers or word processors, as well as for black-and-white or colour portable television sets which have a relatively large number of picture elements. Thus liquid crystal display devices may be used in place of cathode ray tubes. The range of application of liquid crystal display devices whilst already wide is expected to become broader. Accompanying this, it is expected that the properties required for liquid crystal material will change, and the following properties are fundamental and indispensable:
(1) Suffering from no colouration and thermally, optically, electrically and chemically stable;
(2) Wide temperature range for practical use;
(3) Fast electro-optical response time;
(4) Low driving voltage;
(5) Sharp rising in voltage light transmittance and less temperature dependency of threshold voltage (hereinafter referred to as Vₜₕ); and
(6) Wide range of viewing angle.

Although many liquid crystal compounds which are capable of satisfying property (1) are known, there is no known liquid crystal compound capable of satisfying all the properties (2) to (6). In view of the above, a liquid crystal composition comprising a mixture of several kinds of nematic liquid crystal compounds or non-liquid crystal compounds has been used in order to obtain these properties. To satisfy property (4), the threshold voltage has to be lowered. However, since there is the following relationship between Vₜₕ, elastic constants (hereinafter simply referred to as K) and Δε, lowering of Vₜₕ necessitates a liquid crystal compound having great Δε and small K.${\text{V}}_{\text{th}} {\text{∝ (K/Δε )}}^{\text{1/2}}$

The present invention seeks to provide a pyrimidine compound which, when incorporated in a liquid crystal composition, with one or more other nematic liquid crystal compounds or non-liquid crystal compounds, provides a low threshold voltage.

A pyrimidine compound according to the present invention can be obtained by a process illustrated in the following reaction scheme:

### (Step 1) :

Compound (2) and bromine are reacted in chloroform to obtain compound (3).

### (Step 2) :

Compound (3) is reacted with copper (I) cyanide in N-methylpyrrolidone to obtain compound (4).

### (Step 3) :

Compound (4) is reacted with sodium nitrite and sulphuric acid in acetic acid to obtain a diazonium salt and then reacted with copper (I) bromide in hydrobromic acid to obtain compound (5).

### (Step 4) :

Compound (5) is reacted with anhydrous hydrogen chloride gas in a mixed solvent of ethanol and benzene. After distilling off the solvent, the resultant crystals are reacted with anhydrous ammonia gas in ethanol to obtain compound (6).

### (Step 5) :

Compound (6) and compound (7) are reacted in ethanol with metallic sodium to obtain compound (8).

### (Step 7) :

Compound (8) is reacted with copper (I) cyanide in N-methylpyrrolidone to obtain a pyrimidine compound (1) according to the present invention.

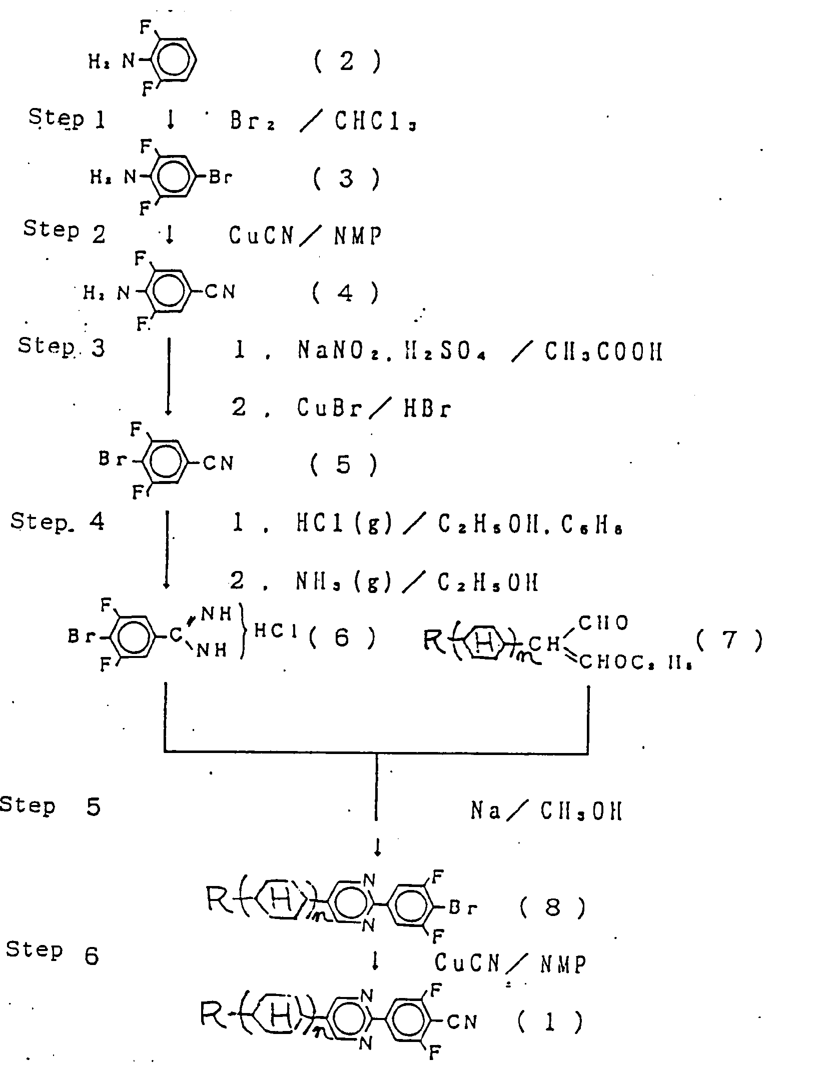

The present invention will now be described in more detail with reference to the following Examples.

### EXAMPLE 1

### Preparation of 2-(3',5'-difluoro-4'cyanophenyl)-5-pentyl pyrimidine.

### (Step 1) :

231 g of 2,6-difluoroaniline was dissolved in 400 ml of chloroform, to which 300 g of bromine was added dropwise. After refluxing for one hour, the reaction solution was poured into an aqueous 10% solution of potassium hydroxide, which was extracted with chloroform and the organic layer was washed with an aqueous 10% solution of potassium hydroxide and water. After distilling off the chloroform, the residue was distilled under reduced pressure (bp: 70 - 80°C/4 mmHg) and re-crystallised from hexane to obtain 292 g of 4-bromo-2,6-difluoroaniline.

### (Step 2) :

132 g of 4-bromo-2,6-difluoroaniline, 70 g of copper (I) cyanide and 444 ml of N-methylpyrrolidone were taken in a flask and refluxed for three hours. Then, the reaction solution was poured into a solution comprising 266 g of iron (III) chloride, 85 ml of concentrated hydrochloric acid and 315 ml of water. The solution was extracted with chloroform and, after washing with water and an aqueous 10% solution of potassium hydroxide, the chloroform was distilled off. The residue was distilled under reduced pressure (bp: 90 - 110°C/4 mmHg) and re-crystallised from a mixed solvent of hexane and chloroform, to obtain 56 g of 4-cyano-2,6-difluoroaniline.

### (Step 3) :

24 g of sodium nitrite was added to 180 ml of concentrated sulphuric acid and, after cooling to a temperature lower than 10°C, 38 ml of acetic acid was added. Then, 53 g of 4-cyano-2,6-difluoroaniline was gradually added so as to keep the temperature of the solution at 20 - 25°C. After stirring at 20 - 25°C for one hour, 60 g of copper (I) bromide was dissolved into 180 ml of an aqueous 48% solution of hydrobromic acid, to which the reaction solution was added dropwise. After the addition was completed, the solution was stirred at room temperature for fifteen hours. After adding water to the reaction solution, it was extracted with chloroform and then washed with water. Chloroform in the aqueous layer was distilled off and the residue was re-crystallised from a mixed solution of methanol and acetone, to obtain 25 g of 2-bromo-5-cyano-1,3-difluorobenzene.

### (Step 4) :

25 g of 2-bromo-5-cyano-1,3-difluorobenzene was dissolved in a mixed solvent of 48 ml of ethanol and 165 ml of benzene and, after cooling to lower than 0°C, anhydrous hydrogen chloride gas was caused to be absorbed for one hour. Then, the solvent of the reaction solution was distilled off and the residual crystals were washed with ether. The resultant crystals were added to 48 ml of ethanol, stirred and then cooled to lower than 0°C. After adding ethanol absorbing 16% ammonia, they were stirred for one hour. The solvent of the reaction solution was distilled off and the residual crystals were washed with ether to obtain 27 g of 4-bromo-3,5-difluorobenzamidine hydrogen chloride.

### (Step 5) :

2.8 g of metallic sodium was dissolved into 220 ml of ethanol to prepare a sodium ethylate solution. Into the solution, 4 g of 4-bromo-3,5-difluorobenzamidine hydrogen chloride and 4.7 g of 3-ethoxy-2-pentyl-acrolein (for the synthesis method, refer to Chem. Ber. 104, 665 to 667 (1971)). Then, after stirring at room temperature over night, the mixture was refluxed for one hour. Then, after distilling off the ethanol, chloroform was added and washed with water and the chloroform was then distilled off. The residue was distilled under reduced pressure (bp: 140 - 150°C/2 mmHg) and then purified by silica gel chloroform column chromatography, to obtain 0.7 g of 2-(3',5'-difluoro-4'-bromophenyl)-5-pentyl pyrimidine.

### (Step 6) :

0.7 g of 2-(3',5'-difluoro-4'-bromophenyl)-5-pentyl pyrimidine, 0.5 g of copper (I) cyanide and 3.5 ml of N-methylpyrrolidone were taken into a flask and then refluxed for five hours. Then, the reaction solution was poured into a solution comprising 1.7 g of iron (III) chloride, 0.4 ml of concentrated hydrochloric acid and 1.7 ml of water. The solution was extracted with chloroform, washed with water and with an aqueous 10% solution of potassium hydroxide and then the chloroform was distilled off. The residue was purified by silica gel chloroform column chromatography and re-crystallised from methanol, to obtain 0.25 g of 2-(3',5'-difluoro-4'-cyanophenyl)-5-pentyl pyrimidine. The crystal - isotropic liquid transfer point (hereinafter simply referred to as C-I point) of this compound was 65.3°C.

Using a similar procedure, the following compounds were prepared by changing the alkyl group in the acrolein compound used in Step 5:
2-(3',5'-difluoro-4'-cyanophenyl)-5-methyl pyrimidine,
2-(3',5'-difluoro-4'-cyanophenyl)-5-ethyl pyrimidine,
2-(3',5'-difluoro-4'-cyanophenyl)-5-propyl pyrimidine,
2-(3',5'-difluoro-4'-cyanophenyl)-5-butyl pyrimidine,
C-I point : 79.3°C
2-(3',5'-difluoro-4'-cyanophenyl)-5-hexyl pyrimidine,
2-(3',5'-difluoro-4'-cyanophenyl)-5-heptyl pyrimidine,
2-(3',5'-difluoro-4'-cyanophenyl)-5-octyl pyrimidine,
2-(3',5'-difluoro-4'-cyanophenyl)-5-nonyl pyrimidine,
2-(3',5'-difluoro-4'-cyanophenyl)-5-decyl pyrimidine.

### EXAMPLE 2

### Preparation of 2-(3',5'-difluoro-4'-cyanophenyl)-5-(trans-4''-propylcyclohexyl) pyrimidine.

Steps 1 to 4 are the same as those in Example 1.

### (Step 5) :

3.7 g of metallic sodium were dissolved into 290 ml of ethanol to prepare a sodium ethylate solution. Into the solution 4 g of 4-bromo-3,5-difluorobenzamidine hydrogen chloride and 8 g of 3-ethoxy-2-(trans-4-propylcyclohexyl)-acrolein (for the synthesis method, refer to A. Villiger, Z. Naturforsch. 34b, 1535 (1979)). Then, after stirring at room temperature over night, the mixture was refluxed for one hour. After cooling the reaction solution, water was added and the deposited crystals were collected by filtration. The resultant crystals were re-crystallised from a mixed solvent of acetone and methanol, the obtain 1.5 g of 2-(3',5'-difluoro-4'-bromophenyl)-5-(trans-4''-propylcyclohexyl) pyrimidine.

### (Step 6) :

1.5 g of 2-(3',5'-difluoro-4'-bromophenyl)-5-(trans-4''-propyl-cyclohexyl) pyrimidine and 0.8 g of copper (I) cyanide and 10 ml of N-methylpyrrolidone were taken into a flask and then refluxed for five hours. Then, the reaction solution was poured into a solution comprising 2.2 g of iron (II) chloride, 0.5 ml of concentrated hydrochloric acid and 2.2 ml of water. The solution was extracted with chloroform, washed with water and with an aqueous 10% solution of potassium hydroxide and then the chloroform was distilled off. The residue was crystallised by silica gel chloroform column chromatography and re-crystallised from a mixed solvent of acetone and methanol, to obtain 0.4 g of 2-(3',5'-difluoro-4'-cyanophenyl)-5-(trans-4''-propylcyclohexyl) pyrimidine. The compound had a nematic liquid crystal phase, C - N point, of 114.9°C and N - I point of 156.7°C.

Using a similar procedure, the following compounds were prepared by changing the alkyl group in the acrolein compound used in Step 5:
2-(3',5'-difluoro-4'-cyanophenyl)-5-(trans-4''-methylcyclohexyl) pyrimidine,
2-(3',5'-difluoro-4'-cyanophenyl)-5-(trans-4''-ethylcyclohexyl) pyrimidine,
2-(3',5'-difluoro-4'-cyanophenyl)-5-(trans-4''-butylcyclohexyl) pyrimidine,
2-(3',5'-difluoro-4'-cyanophenyl)-5-(trans-4''-pentylcyclohexyl) pyrimidine,
2-(3',5'-difluoro-4'-cyanophenyl)-5-(trans-4''-hexylcyclohexyl) pyrimidine,
2-(3',5'-difluoro-4'-cyanophenyl)-5-(trans-4''-heptylcyclohexyl) pyrimidine,
2-(3',5'-difluoro-4'-cyanophenyl)-5-(trans-4''-octylcyclohexyl) pyrimidine,
2-(3',5'-difluoro-4'-cyanophenyl)-5-(trans-4''-nonylcyclohexyl) pyrimidine,
2-(3',5'-difluoro-4'-cyanophenyl)-5-(trans-4''-decylcyclohexyl) pyrimidine.

### EXAMPLE 3

Using a commercially available mixed liquid crystal material ZLI-1565 (product manufactured by Merk Co. N - I point, 89.3°C) as the base, Composition A consisting of this base and 2-(4'-cyanophenyl)-5-pentyl pyrimidine generally used at present with a view to lowering Vₜₕ and Composition B consisting of this base and 2-(3',5'-difluoro-4'-cyanophenyl)-5-pentyl pyrimidine of Example 1 were compared as regards their properties.

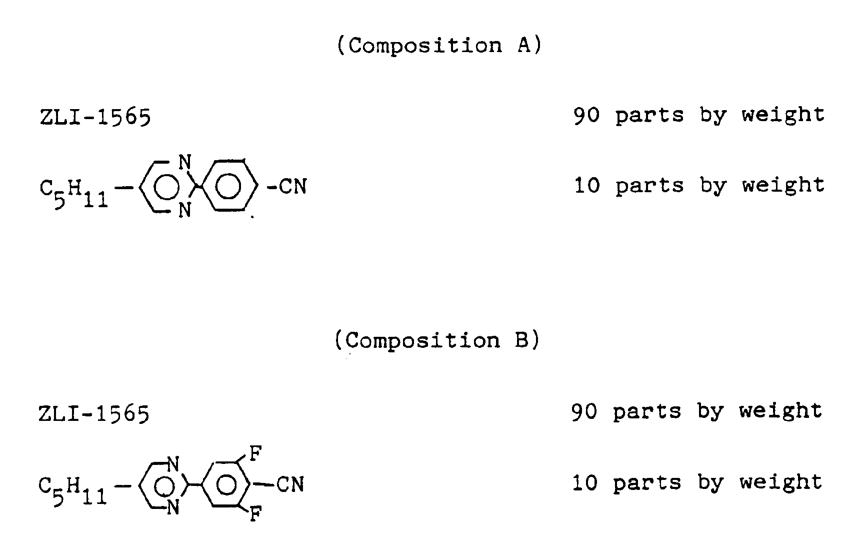

Compositions A and B were each sealed in a TN cell of 7 µm thickness and voltage-transmittance characteristics were measured under AC static driving at 20°C. The results are given in Table 1.

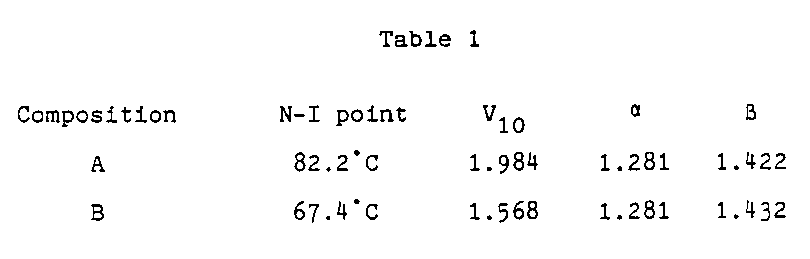

V₁₀ in Table 1 is a voltage value at 10°C transmittance measured at an angle of ϑ = 90° for the TN cell. α and β represent, respectively, view angle property and threshold property, which are defined respectively as:-$\text{α = ϑ90°V₅₀/ϑ50°V₅₀}$$\text{β = ϑ90°V₁₀/ϑ90°V₉₀}$

### EXAMPLE 4

Using a commercially available mixed liquid crystal material ZLI-1565 (product manufactured by Merk Co. N - I point, 89.3°C) as the base, Composition C consisting of this base and 4-pentyl-4''-cyanoterphenyl used generally at present with a view to increasing the N - I point and Composition D consisting of this base and 2-(3',5'-difluoro-4'-cyanophenyl)-5-(trans-4''-pentylcyclohexyl) pyrimidine of Example 1 were compared as regards their properties.

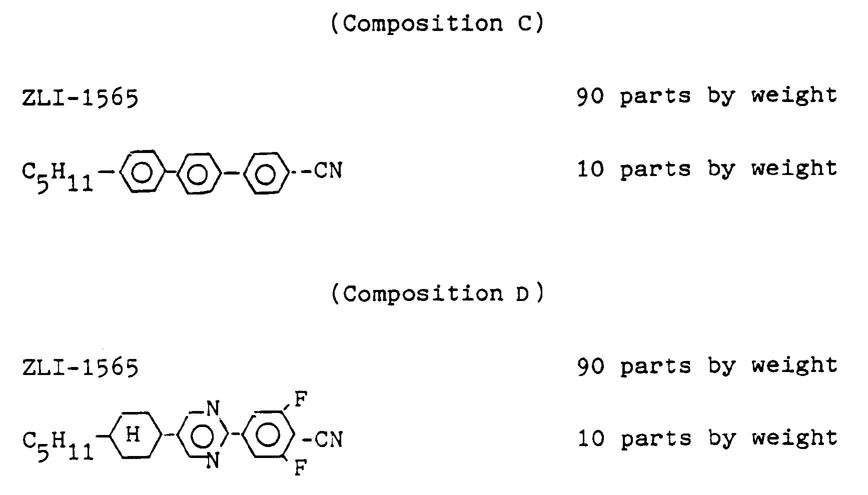

Compositions C and D were each sealed in a TN cell of 7 µm thickness and voltage-transmittance characteristics were measured under AC static driving at 20°C. The results are given in Table 2.

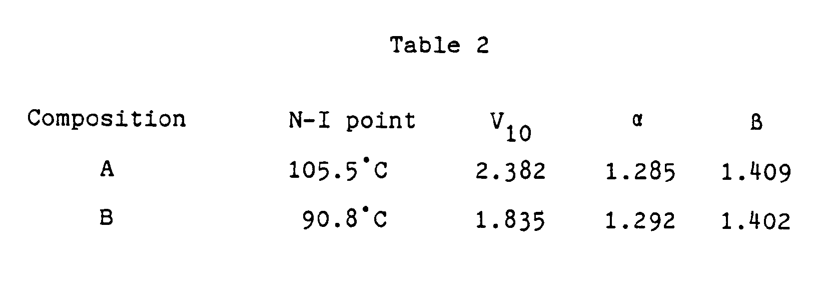

As has been described above, a pyrimidine compound according to the present invention when mixed with, for example, a conventional liquid crystal composition, can greatly lower the threshold voltage without worsening the electro-optical properties (α value and β value). Thus the present invention is extremely useful as the basic component of a liquid crystal composition for super twisted nematic type display devices which are the predominant type of liquid crystal display device currently in use.

## Claims

1. A pyrimidine derivative of the general formula: 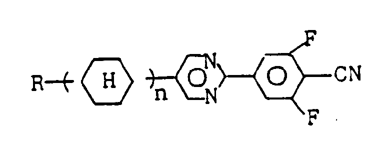 where R is a straight chain alkyl group with 1 to 10 carbon atoms, n is 0 or 1 and the cyclohexane ring is in the trans form.

2. 2-(3',5'-difluoro-4'-cyanophenyl)-5-pentyl pyrimidine, or
2-(3',5'-difluoro-4'-cyanophenyl)-5-methyl pyrimidine, or
2-(3',5'-difluoro-4'-cyanophenyl)-5-ethyl pyrimidine, or
2-(3',5'-difluoro-4'-cyanophenyl)-5-propyl pyrimidine,
or
2-(3',5'-difluoro-4'-cyanophenyl)-5-butyl pyrimidine, or
2-(3',5'-difluoro-4'-cyanophenyl)-5-hexyl pyrimidine, or
2-(3',5'-difluoro-4'-cyanophenyl)-5-heptyl pyrimidine,
or
2-(3',5'-difluoro-4'-cyanophenyl)-5-octyl pyrimidine, or
2-(3',5'-difluoro-4'-cyanophenyl)-5-nonyl pyrimidine, or
2-(3',5'-difluoro-4'-cyanophenyl)-5-decyl pyrimidine.

3. 2-(3',5'-difluoro-4'-cyanophenyl)-5-(trans-4''-propylcyclohexyl) pyrimidine, or
2-(3',5'-difluoro-4'-cyanophenyl)-5-(trans-4''-methylcyclohexyl) pyrimidine, or
2-(3',5'-difluoro-4'-cyanophenyl)-5-(trans-4''-ethylcyclohexyl) pyrimidine, or
2-(3',5'-difluoro-4'-cyanophenyl)-5-(trans-4''-butylcyclohexyl) pyrimidine, or
2-(3',5'-difluoro-4'-cyanophenyl)-5-(trans-4''-pentylcyclohexyl) pyrimidine, or
2-(3',5'-difluoro-4'-cyanophenyl)-5-(trans-4''-hexylcyclohexyl) pyrimidine, or
2-(3',5'-difluoro-4'-cyanophenyl)-5-(trans-4''-heptylcyclohexyl) pyrimidine, or
2-(3',5'-difluoro-4'-cyanophenyl)-5-(trans-4''-octylcyclohexyl) pyrimidine, or
2-(3',5'-difluoro-4'-cyanophenyl)-5-(trans-4''-nonylcyclohexyl) pyrimidine, or
2-(3',5'-difluoro-4'-cyanophenyl)-5-(trans-4''-decylcyclohexyl) pyrimidine.

## Patentansprüche

1. Ein Pyrimidinderivat der allgemeinen Formel: 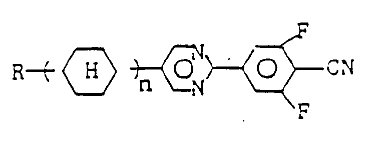 in der R eine geradkettige Alkylgruppe mit 1 bis 10 Kohlenstoffatomen ist, n 0 oder 1 ist und der Cyclohexanring in der trans-Form ist.

2. 2-(3',5'-Difluor-4'-cyanphenyl)-5-pentylpyrimidin, oder
2-(3',5'-Difluor-4'-cyanphenyl)-5-methylpyrimidin, oder
2-(3',5'-Difluor-4'-cyanphenyl)-5-ethylpyrimidin, oder
2-(3',5'-Difluor-4'-cyanphenyl)-5-propylpyrimidin,
oder
2-(3',5'-Difluor-4'-cyanphenyl)-5-butylpyrimidin, oder
2-(3',5'-Difluor-4'-cyanphenyl)-5-hexylpyrimidin, oder
2-(3',5'-Difluor-4'-cyanphenyl)-5-heptylpyrimidin,
oder
2-(3',5'-Difluor-4'-cyanphenyl)-5-octylpyrimidin, oder
2-(3',5'-Difluor-4'-cyanphenyl)-5-nonylpyrimidin, oder
2-(3',5'-Difluor-4'-cyanphenyl)-5-decylpyrimidin.

3. 2-(3',5'-Difluor-4'-cyanphenyl)-5-(trans-4''-propylcyclohexyl)pyrimidin, oder
2-(3',5'-Difluor-4'-cyanphenyl)-5-(trans-4''-methylcyclohexyl)pyrimidin, oder
2-(3',5'-Difluor-4'-cyanphenyl)-5-(trans-4''-ethylcyclohexyl)pyrimidin, oder
2-(3',5'-Difluor-4'-cyanphenyl)-5-(trans-4''-butylcyclohexyl)pyrimidin, oder
2-(3',5'-Difluor-4'-cyanphenyl)-5-(trans-4''-pentylcyclohexyl)pyrimidin, oder
2-(3',5'-Difluor-4'-cyanphenyl)-5-(trans-4''-hexylcyclohexyl)pyrimidin, oder
2-(3',5'-Difluor-4'-cyanphenyl)-5-(trans-4''-heptylcyclohexyl)pyrimidin, oder
2-(3',5'-Difluor-4'-cyanphenyl)-5-(trans-4''-octylcyclohexyl)pyrimidin, oder
2-(3',5'-Difluor-4'-cyanphenyl)-5-(trans-4''-nonylcyclohexyl)pyrimidin, oder
2-(3',5'-Difluor-4'-cyanphenyl)-5-(trans-4''-decylcyclohexyl)pyrimidin.

## Revendications

1. Dérivé de la pyrimidine répondant à la formule générale 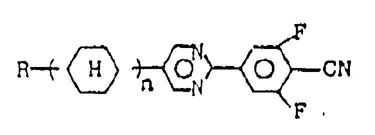 dans laquelle R est un groupe alkyle à chaîne droite ayant de 1 à 10 atomes de carbone, n vaut 0 ou 1 et le noyau cyclohexane se présente sous la forme trans.

2. 2-(3',5'-difluoro-4'-cyanophényl)-5-pentyl-pyrimidine, ou
2-(3',5'-difluoro-4'-cyanophényl)-5-méthyl-pyrimidine, ou
2-(3',5'-difluoro-4'-cyanophényl)-5-éthyl-pyrimidine, ou
2-(3',5'-difluoro-4'-cyanophényl)-5-propyl-pyrimidine, ou
2-(3',5'-difluoro-4'-cyanophényl)-5-butyl-pyrimidine, ou
2-(3',5'-difluoro-4'-cyanophényl)-5-hexyl-pyrimidine, ou
2-(3',5'-difluoro-4'-cyanophényl)-5-heptyl-pyrimidine, ou
2-(3',5'-difluoro-4'-cyanophényl)-5-octyl-pyrimidine, ou
2-(3',5'-difluoro-4'-cyanophényl)-5-nonyl-pyrimidine, ou
2-(3',5'-difluoro-4'-cyanophényl)-5-décyl-pyrimidine.

3. 2-(3',5'-difluoro-4'-cyanophényl)-5-(trans-4''-propylcyclohexyl)-pyrimidine, ou
2-(3',5'-difluoro-4'-cyanophényl)-5-(trans-4''-méthylcyclohexyl)-pyrimidine, ou
2-(3',5'-difluoro-4'-cyanophényl)-5-(trans-4''-éthylcyclohexyl)-pyrimidine, ou
2-(3',5'-difluoro-4'-cyanophényl)-5-(trans-4''-butylcyclohexyl)-pyrimidine, ou
2-(3',5'-difluoro-4'-cyanophényl)-5-(trans-4''-pentylcyclohexyl)-pyrimidine, ou
2-(3',5'-difluoro-4'-cyanophényl)-5-(trans-4''-hexylcyclohexyl)-pyrimidine, ou
2-(3',5'-difluoro-4'-cyanophényl)-5-(trans-4''-heptylcyclohexyl)-pyrimidine, ou
2-(3',5'-difluoro-4'-cyanophényl)-5-(trans-4''-octylcyclohexyl)-pyrimidine, ou
2-(3',5'-difluoro-4'-cyanophényl)-5-(trans-4''-nonylcyclohexyl)-pyrimidine, ou
2-(3',5'-difluoro-4'-cyanophényl)-5-(trans-4''-décylcyclohexyl)-pyrimidine.
